# EUROPEAN PATENT APPLICATION

(11) **EP 3 205 350 A1**
(43) Date of publication of application: **16.08.2017**
(21) Application number: 16201169.6
(22) Date of filing: 14.12.2011
(51) Int. Cl.: A61K 38/40, A23L 33/10, A23L 33/19, A61P 37/04

(54) **INFANT FORMULAS INCLUDING LACTOFERRIN FOR STIMULATING IMMUNE CELLS**

(30) Priority: 29.12.2010 US 201013980819
(62) Divisional of application: 11804622.6
(71) Applicant: MJN U.S. Holdings, LLC, Glenview, IL 60026 (US)
(72) Inventor: Wittke, Anja, Evansville, IN Indiana 47714 (US); Munoz, Cecilia, Westerville, OH Ohio 43082 (US); Banavara, Dattatreya, Newburgh, IN Indiana 47630 (US)
(74) Representative: V.O.

(57) **Abstract**

The present disclosure relates to infant formulas including lactoferrin produced by a non-human source, for use in stimulating innate immune cells, such as macrophages, neutrophils, and dendritic cells.

## Description

### TECHNICAL FIELD

This disclosure relates generally to the field of nutritional compositions, such as infant formulas, human milk fortifiers, children's dietary supplements, and the like, having lactoferrin, in particular lactoferrin produced by a non-human source. More particularly, the disclosure relates to a method of stimulating innate immune cells in a human by administering to the human nutritional compositions including lactoferrin produced by a non-human source.

### BACKGROUND ART

There are currently a variety of dietary compositions for humans, especially young humans, to provide supplemental or primary nutrition at certain stages in life. Generally, commercial dietary compositions for infants seek to mimic to the extent possible the composition and associated functionality of human milk. Through a combination of proteins, some of which have physiological activity, and blended fat ingredients, dietary compositions are formulated such that they simulate human milk for use as a complete or partial substitute. Other ingredients often utilized in dietary compositions for infants may include a carbohydrate source such as lactose as well as other vitamins, minerals and elements believed to be present in human milk for the absorption by the infant.

Lactoferrin is one of the primary proteins in human milk and is considered a glycoprotein having an average molecular weight of approximately 80 kilodaltons. It is an iron binding protein having the capacity to bind two molecules of iron in a reversible fashion and can facilitate the uptake of iron within the intestines for the human. Functionally, lactoferrin regulates iron absorption and as such can bind iron-based free radicals as well as donate iron for an immunological response.

An additional role of lactoferrin is its anti-microbial activity in guarding against intestinal infections in humans generally, but especially in infants. Lactoferrin has been known to be both bacteriostatic and bactericidal in inhibiting the growth of specific bacteria while also killing microbes prior to a successful invasion of intestinal cells.

In obtaining a commercially viable dietary composition, the addition of lactoferrin has generally been limited due to predicted losses of activity during processing. For example, generally, the temperature and pH requirements in processing infant formulas and other products such as human milk fortifiers and various children's products reduce specific functions of the lactoferrin, causing lactoferrin not to be included within a final formulation. In addition, lactoferrin is often considered only for its iron binding qualities; thus, lactoferrin may generally be excluded from a formulation where such properties are thought to be diminished by processing conditions.

Further, as known in the art, human breast milk is relatively low in iron, containing about 0.3 milligrams of iron per liter of breast milk. While this quantity is low, human infants have high absorption rate, absorbing about half of the iron from the breast milk. However, when human infants are given prior art formulas with high levels of iron fortification, for example, of from about 10 mg to about 12 milligrams per liter, the infants absorb less than about 5% of the total iron. With such increased levels of iron within the prior art formulas, virtually all of the iron binding sites would be expected to be occupied, as lactoferrin is a known iron transport protein.

Additional complications of the prior art formulas include the inability of providing a bacteriostatic effect. This is partially due to the use of lactoferrin with blocked or damaged binding sites, as the bacteriostatic effect is at least partially related to the degree of binding to iron of the lactoferrin present within the formula.

Accordingly, it would be beneficial to provide a nutritional composition, such as an infant formula, human milk fortifier, children's dietary supplement, and the like, which contains lactoferrin, in particular, lactoferrin produced by a non-human source. Preferably, the lactoferrin included in the compositions is able to stimulate innate immune cells even after processing under conditions of high temperature and low pH. It also would be beneficial to determine the response of innate immune cells to nutritional compositions including lactoferrin.

### DISCLOSURE OF THE INVENTION

Briefly, the present disclosure is directed, in an embodiment, to a method for stimulating at least one type of innate immune cell in a human. In certain embodiments, the method comprises administering to the human a nutritional composition comprising:
a. up to about 7 g/100 kcal of a fat or lipid source, more preferably about 3 g/100 kcal to about 7 g/100 kcal of a fat or lipid source;
b. up to about 5 g/100 kcal of a protein source, more preferably about 1 g/100 kcal to about 5 g/100 kcal of a protein source; and
c. at least about 10 mg/100 kCal of lactoferrin, more preferably about 70 mg to about 220 mg/100 kCal of lactoferrin, and most preferably about 90 mg to about 190 mg/100 kCal of lactoferrin. Optionally, in certain embodiments, the nutritional compositions may further comprise about 0.1 g/100 kcal to about 1 g/100 kcal of a prebiotic composition comprising polydextrose and/or galactooligosaccharide. More preferably, the nutritional composition comprises about 0.3 g/100 kcal to about 0.7 g/100 kcal of a prebiotic composition which comprises a combination of polydextrose and galactooligosaccharide.

Preferably, the lactoferrin is non-human lactoferrin and/or human lactoferrin produced by a genetically modified organism. In one particularly preferred embodiment, the lactoferrin used is such that an effective amount of a nutritional composition containing lactoferrin may be administered to stimulate at least one type of innate immune cell in a human, even if, during processing, the nutritional composition has been exposed to pH and temperature fluctuations typical of certain processing conditions like pasteurization.

### BEST MODE FOR CARRYING OUT THE INVENTION

In certain embodiments, the present disclosure provides a method for stimulating at least one type of innate immune cell in a human by administering to the human nutritional compositions that comprise a lipid or fat source, a protein source, and lactoferrin produced by a non-human source.

As used herein, "lactoferrin from a non-human source" means lactoferrin which is produced by or obtained from a source other than human breast milk. For example, lactoferrin for use in the present disclosure includes human lactoferrin produced by a genetically modified organism as well as non-human lactoferrin. The term "organism", as used herein, refers to any contiguous living system, such as animal, plant, fungus or micro-organism. The term "non-human lactoferrin", as used herein, refers to lactoferrin having an amino acid sequence that is different than the amino acid sequence of human lactoferrin.

Lactoferrins are single chain polypeptides of about 80 kD containing 1 - 4 glycans, depending on the species. The 3-D structures of lactoferrin of different species are very similar, but not identical. Each lactoferrin comprises two homologous lobes, called the N- and C-lobes, referring to the N-terminal and C-terminal part of the molecule, respectively. Each lobe further consists of two sub-lobes or domains, which form a cleft where the ferric ion (Fe³⁺) is tightly bound in synergistic cooperation with a (bi)carbonate anion. These domains are called N1, N2, C1 and C2, respectively. The N-terminus of lactoferrin has strong cationic peptide regions that are responsible for a number of important binding characteristics. Lactoferrin has a very high isoelectric point (∼pI 9) and its cationic nature plays a major role in its ability to defend against bacterial, viral, and fungal pathogens. There are several clusters of cationic amino acids residues within the N-terminal region of lactoferrin mediating the biological activities of lactoferrin against a wide range of microorganisms. For instance, the N-terminal residues 1-47 of human lactoferrin (1-48 of bovine lactoferrin) are critical to the iron-independent biological activities of lactoferrin. In human lactoferrin, residues 2 to 5 (RRRR) and 28 to 31 (RKVR) are arginine-rich cationic domains in the N-terminus especially critical to the antimicrobial activities of lactoferrin. A similar region in the N-terminus is found in bovine lactoferrin (residues 17 to 42; FKCRRWQWRMKKLGAPSITCVRRAFA).

As described in "Perspectives on Interactions Between Lactoferrin and Bacteria" which appeared in the publication BIOCHEMISTRY AND CELL BIOLOGY, pp 275-281 (2006), lactoferrins from different host species may vary in their amino acid sequences though commonly possess a relatively high isoelectric point with positively charged amino acids at the end terminal region of the internal lobe. Suitable lactoferrins for use in the present disclosure include those having at least 48% homology with the amino acid sequence AVGEQELRKCNQWSGL at the HLf (349-364) fragment. In some embodiments, the lactoferrin has at least 65% homology with the amino acid sequence AVGEQELRKCNQWSGL at the HLf (349-364) fragment, and, in embodiments, at least 75% homology. For example, non-human lactoferrins acceptable for use in the present disclosure include, without limitation, bovine lactoferrin, porcine lactoferrin, equine lactoferrin, buffalo lactoferrin, goat lactoferrin, murine lactoferrin and camel lactoferrin.

Lactoferrin for use in the present disclosure may be, for example, isolated from the milk of a non-human animal or produced by a genetically modified organism. For example, in U.S. Patent No. 4,791,193, incorporated by reference herein in its entirety, Okonogi et al. discloses a process for producing bovine lactoferrin in high purity. Generally, the process as disclosed includes three steps. Raw milk material is first contacted with a weakly acidic cationic exchanger to absorb lactoferrin followed by the second step where washing takes place to remove nonabsorbed substances. A desorbing step follows where lactoferrin is removed to produce purified bovine lactoferrin. Other methods may include steps as described in U.S. Patent Nos. 7,368,141, 5,849,885, 5,919,913 and 5,861,491, the disclosures of which are all incorporated by reference in their entirety.

In one embodiment, lactoferrin is present in the nutritional composition in an amount of at least about 10 mg/100kCal, especially when the nutritional composition is intended for use by children. In certain embodiments, the upper limit for lactoferrin is about 240 mg/100 kCal. In another embodiment, where the nutritional composition is an infant formula, lactoferrin is present in the nutritional composition in an amount of from about 70 mg to about 220 mg/100 kCal; in yet another embodiment, lactoferrin is present in an amount of about 90 mg to about 190 mg/100 kCal. Nutritional compositions for infants can include lactoferrin in the quantities of from about 0.5 mg to about 1.5 mg per milliliter of formula. In nutritional compositions replacing human milk, lactoferrin may be present in quantities of from about 0.6 mg to about 1.3 mg per milliliter of formula.

As mentioned, in certain embodiments of the disclosure, the nutritional compositions stimulate at least one type of innate immune cell when administered to humans. Several types of innate immune cells are well-known in the art and include, without, limitation, natural killer cells, mast cells, eosinophils, basophils; and the phagocytic cells including macrophages, neutrophils and dendritic cells. Preferably, when administered to humans, the nutritional compositions stimulate macrophages, dendritic cells and/or neutrophils. The nutritional compositions may stimulate the innate immune cells by any means known in the art. Preferably, the nutritional compositions stimulate the expression of costimulatory molecules from macrophages and/or dendritic cells. In another preferred embodiment, the nutritional compositions stimulate the production of cytokines from macrophages and/or dendtritic cells. In yet another preferred embodiment, the nutritional compositions stimulate the respiratory burst capacity of neutrophils.

Preferably, the lactoferrin used in the nutritional composition retains its stability and activity in the human gut even after processing under conditions of high temperature and low pH. In one embodiment of the present disclosure, the lactoferrin used is non-human lactoferrin.

For example, surprisingly, bovine lactoferrin maintains certain bactericidal activity even if exposed to a low pH (i.e., below 7, and even as low as about 4.6 or lower) and/or high temperatures (i.e., above about 65°C, and as high as about 120°C), conditions which would be expected to destroy or severely limit the stability or activity of human lactoferrin. These low pH and/or high temperature conditions can be expected during certain processing regimen for nutritional compositions of the types described herein, such as pasteurization. Yet, while bovine lactoferrin has an amino acid composition which has about a 70% sequence homology to that of human lactoferrin, and is stable and remains active under conditions under which human lactoferrin becomes unstable or inactive, bovine lactoferrin has bactericidal activity against undesirable bacterial pathogens found in the human gut.

In some embodiments, the nutritional compositions of the disclosure may be an infant formula. The term "infant formula" applies to a composition in liquid or powdered form that satisfies the nutrient requirements of an infant by being a substitute for human milk. In the United States, the content of an infant formula is dictated by the federal regulations set forth at 21 C.F.R. §§100, 106 and 107. These regulations define macronutrient, vitamin, mineral, and other ingredient levels in an effort to simulate the nutritional and other properties of human breast milk. In a separate embodiment, the nutritional product may be a human milk fortifier, meaning it is a composition which is added to human milk in order to enhance the nutritional value of human milk. As a human milk fortifier, the disclosed composition may be in powder or liquid form. In yet another embodiment, the disclosed nutritional product may be a children's nutritional composition.

Preferably, the nutritional composition is administered to an infant or a child. As used herein, the term "infant" is generally defined as a human from birth to 12 months of age. A "child" and "children" are defined as humans over the age of 12 months to about 12 years old.

The nutritional compositions of the disclosure may provide minimal, partial, or total nutritional support. The nutritional compositions may be nutritional supplements or meal replacements. In some embodiments, the nutritional compositions may be administered in conjunction with a food or another nutritional composition. In this embodiment, the nutritional compositions can either be intermixed with the food or other nutritional composition prior to ingestion by the subject or can be administered to the subject either before or after ingestion of a food or nutritional composition. The nutritional compositions may be administered to preterm infants receiving infant formula, breast milk, a human milk fortifier, or combinations thereof. For purposes of the present disclosure, a "preterm infant" is an infant born after less than 37 weeks gestation, while a "full term infant" is an infant born after at least 37 weeks gestation.

The nutritional compositions may, but need not, be nutritionally complete. The skilled artisan will recognize "nutritionally complete" to vary depending on a number of factors including, but not limited to, age, clinical condition, and dietary intake of the subject to whom the term is being applied. In general, "nutritionally complete" means that the nutritional composition of the present disclosure provides adequate amounts of all carbohydrates, lipids, essential fatty acids, proteins, essential amino acids, conditionally essential amino acids, vitamins, minerals, and energy required for normal growth. As applied to nutrients, the term "essential" refers to any nutrient which cannot be synthesized by the body in amounts sufficient for normal growth and to maintain health and which therefore must be supplied by the diet. The term "conditionally essential" as applied to nutrients means that the nutrient must be supplied by the diet under conditions when adequate amounts of the precursor compound is unavailable to the body for endogenous synthesis to occur.

The composition which is "nutritionally complete" for the preterm infant will, by definition, provide qualitatively and quantitatively adequate amounts of all carbohydrates, lipids, essential fatty acids, proteins, essential amino acids, conditionally essential amino acids, vitamins, minerals, and energy required for growth of the preterm infant. The composition which is "nutritionally complete" for the full term infant will, by definition, provide qualitatively and quantitatively adequate amounts of all carbohydrates, lipids, essential fatty acids, proteins, essential amino acids, conditionally essential amino acids, vitamins, minerals, and energy required for growth of the full term infant. The composition which is "nutritionally complete" for a child will, by definition, provide qualitatively and quantitatively adequate amounts of all carbohydrates, lipids, essential fatty acids, proteins, essential amino acids, conditionally essential amino acids, vitamins, minerals, and energy required for growth of a child.

The nutritional composition may be provided in any form known in the art, including a powder, a gel, a suspension, a paste, a solid, a liquid, a liquid concentrate, or a ready-to-use product. In one preferred embodiment, the nutritional composition is an infant formula, especially an infant formula adapted for use as sole source nutrition for an infant.

In the preferred embodiments, the nutritional product disclosed herein may be administered enterally. As used herein, "enteral" means through or within the gastrointestinal, or digestive, tract, and "enteral administration" includes oral feeding, intragastric feeding, transpyloric administration, or any other introduction into the digestive tract.

Suitable fat or lipid sources for practicing the present disclosure may be any known or used in the art, including but not limited to, animal sources, e.g., milk fat, butter, butter fat, egg yolk lipid; marine sources, such as fish oils, marine oils, single cell oils; vegetable and plant oils, such as corn oil, canola oil, sunflower oil, soybean oil, palmolein, coconut oil, high oleic sunflower oil, evening primrose oil, rapeseed oil, olive oil, flaxseed (linseed) oil, cottonseed oil, high oleic safflower oil, palm stearin, palm kernel oil, wheat germ oil; medium chain triglyceride oils and emulsions and esters of fatty acids; and any combinations thereof.

In certain embodiments, the protein source included in the nutritional composition comprises bovine milk proteins. Bovine milk protein sources useful in practicing the present disclosure include, but are not limited to, milk protein powders, milk protein concentrates, milk protein isolates, nonfat milk solids, nonfat milk, nonfat dry milk, whey protein, whey protein isolates, whey protein concentrates, sweet whey, acid whey, casein, acid casein, caseinate (*e.g*. sodium caseinate, sodium calcium caseinate, calcium caseinate) and any combinations thereof.

In one embodiment, the proteins are provided as intact proteins. In other embodiments, the proteins are provided as a combination of both intact proteins and partially hydrolyzed proteins, with a degree of hydrolysis of between about 4% and 10%. In yet another embodiment, the protein source may be supplemented with glutamine-containing peptides.

In a particular embodiment of the disclosure, the protein source comprises whey and casein proteins and the ratio of whey to casein proteins ratio is similar to that found in human breast milk. For example, in certain embodiments, the weight ratio of whey to casein proteins is from about 20% whey:80% casein to about 80% whey:20% casein.

In one embodiment of the disclosure, the nutritional composition may contain one or more probiotics. The term "probiotic" means a microorganism with low or no pathogenicity that exerts beneficial effects on the health of the host. Any probiotic known in the art may be acceptable in this embodiment provided it achieves the intended result. In a particular embodiment, the probiotic may be selected from *Lactobacillus* species, *Lactobacillus rhamnosus* GG, *Bifidobacterium* species, *Bifidobacterium brevis, Bifidobacterium longum,* and *Bifidobacterium animalis subsp. lactis* BB-12.

If included in the composition, the amount of the probiotic may vary from about 10⁴ to about 10¹⁰ colony forming units (cfu) per kg body weight per day. In another embodiment, the amount of the probiotic may vary from about 10⁶ to about 10⁹ cfu per kg body weight per day. In yet another embodiment, the amount of the probiotic may be at least about 10⁶ cfu per kg body weight per day. Moreover, the disclosed composition may also include probiotic-conditioned media components.

In one embodiment, one or more of the probiotics is viable. In another embodiment, one or more of the probiotics is non-viable. As used herein, the term "viable" refers to live microorganisms. The term "non-viable" or "non-viable probiotic" means non-living probiotic microorganisms, their cellular components and metabolites thereof. Such non-viable probiotics may have been heat-killed or otherwise inactivated but retain the ability to favorably influence the health of the host. The probiotics useful in the present disclosure may be naturally-occurring, synthetic or developed through the genetic manipulation of organisms, whether such new source is now known or later developed.

In one embodiment of the disclosure, the nutritional compositions may include a prebiotic composition comprising one or more prebiotics. As used herein, the term "prebiotic" means a non-digestible food ingredient that beneficially affects the host by selectively stimulating the growth and/or activity of one or a limited number of bacteria in the colon that can improve the health of the host. A "prebiotic composition" is a composition that comprises one or more prebiotics. Such prebiotics may be naturally-occurring, synthetic, or developed through the genetic manipulation of organisms and/or plants, whether such new source is now known or developed later. In certain embodiments, the prebiotic included in the compositions of the present disclosure include those taught by U.S. Patent No. 7,572,474, the disclosure of which is incorporated herein by reference.

Prebiotics useful in the present disclosure may include oligosaccharides, polysaccharides, and other prebiotics that contain fructose, xylose, soya, galactose, glucose and mannose. More specifically, prebiotics useful in the present disclosure may include lactulose, lactosucrose, raffinose, gluco-oligosaccharide, inulin, polydextrose, polydextrose powder, galactooligosaccharide, fructo-oligosaccharide, isomalto-oligosaccharide, soybean oligosaccharides, lactosucrose, xylo-oligosacchairde, chito-oligosaccharide, manno-oligosaccharide, aribino-oligosaccharide, siallyl-oligosaccharide, fuco-oligosaccharide, and gentio-oligosaccharides. Preferably, the nutritional compositions comprise polydextrose and/or galactooligosaccaharide. Optionally, in addition to polydextrose and/or galactooligosaccaharide, the nutritional compositions comprise one or more additional prebiotics.

If included in the nutritional compositions, the total amount of prebiotics present in the nutritional composition may be from about 0.1 g/100 kcal to about 1 g/100 kcal. More preferably, the total amount of prebiotics present in the nutritional composition may be from about 0.3 g/100 kcal to about 0.7 g/100 kcal. At least 20% of the prebiotics should comprise galactooligosaccharide (GOS) and/or polydextrose (PDX).

If polydextrose is used in the prebiotic composition, the amount of polydextrose in the nutritional composition may, in an embodiment, be within the range of from about 0.1 g/100 kcal to about 1 g/100 kcal. In another embodiment, the amount of polydextrose in the nutritional composition is within the range of from about 0.2 g/100 kcal to about 0.6 g/100 kcal.

If galactooligosaccharide is used in the prebiotic composition, the amount of galactooligosaccharide in the nutritional composition may, in an embodiment, be from about 0.1 g/100 kcal to about 1 g/100 kcal. In another embodiment, the amount of galactooligosaccharide in the nutritional composition is from about 0.2 g/100 kcal to about 0.5 g/100 kcal. In certain embodiments, the ratio of polydextrose to galactooligosaccharide in the prebiotic composition is between about 9:1 and about 1:9.

The nutritional formulation of the disclosure, in some embodiments, may further contain a source of long chain polyunsaturated fatty acids (LCPUFAs). Preferably, the source of LCPUFAs comprise docosahexanoic acid (DHA). Other suitable LCPUFAs include, but are not limited to, α-linoleic acid, γ-linoleic acid, linoleic acid, linolenic acid, eicosapentanoic acid (EPA) and arachidonic acid (ARA).

In one embodiment, the nutritional composition is supplemented with both DHA and ARA. In this embodiment, the weight ratio of ARA:DHA may be from about 1:3 to about 9:1. In one embodiment of the present disclosure, the weight ratio of ARA:DHA is from about 1:2 to about 4:1.

The amount of long chain polyunsaturated fatty acids in the nutritional composition may vary from about 5 mg/100 kcal to about 100 mg/100 kcal, more preferably from about 10 mg/100 kcal to about 50 mg/100 kcal.

The nutritional composition may be supplemented with oils containing DHA and ARA using standard techniques known in the art. For example, DHA and ARA may be added to the composition by replacing an equivalent amount of an oil, such as high oleic sunflower oil, normally present in the composition. As another example, the oils containing DHA and ARA may be added to the composition by replacing an equivalent amount of the rest of the overall fat blend normally present in the composition without DHA and ARA.

If utilized, the source of DHA and ARA may be any source known in the art such as marine oil, fish oil, single cell oil, egg yolk lipid, and brain lipid. In some embodiments, the DHA and ARA are sourced from the single cell Martek oil, DHASCO® and ARASCO® respectively, or variations thereof. The DHA and ARA can be in natural form, provided that the remainder of the LCPUFA source does not result in any substantial deleterious effect on the infant. Alternatively, the DHA and ARA can be used in refined form.

In an embodiment of the present disclosure, sources of DHA and ARA are single cell oils as taught in U.S. Pat. Nos. 5,374,567; 5,550,156; and 5,397,591, the disclosures of which are incorporated herein in their entirety by reference. However, the present disclosure is not limited to only such oils.

In certain embodiments, the nutritional compositions comprise from about 0.5 mg/100 kcal to about 5 mg/100 kcal of iron, including iron bound to lactoferrin.

### EXAMPLES

The following examples are provided to illustrate embodiments of the nutritional composition of the present disclosure but should not be interpreted as any limitation thereon. Other embodiments within the scope of the claims herein will be apparent to one skilled in the art from the consideration of the specification or practice of the nutritional composition or methods disclosed herein. It is intended that the specification, together with the example, be considered to be exemplary only, with the scope and spirit of the disclosure being indicated by the claims which follow the examples.

### EXAMPLE 1

This example illustrates an embodiment of a nutritional product according to the present disclosure.

| **Description** | **kg per 100 kg** |
|---|---|
| carbohydrate, total | 38.9 |
| protein, total | 28.8 |
| fat, total | 25.6 |
| | |
| prebiotics | 4.5 |
| soy lecithin | 0.8 |
| lactoferrin | 0.3 |
| calcium carbonate | 0.5 |
| potassium citrate | 0.2 |
| ferrous sulfate | 0.05 |
| potassium chloride | 0.048 |
| magnesium oxide | 0.023 |
| sodium chloride | 0.025 |
| zinc sulfate | 0.015 |
| cupric sulfate | 0.002 |
| manganese sulfate | 0.0003 |
| sodium selenite | 0.00003 |
| choline chloride | 0.144 |
| ascorbic acid | 0.093 |
| Niacinamide | 0.006 |
| calcium pantothenate | 0.003 |
| vitamin A palmitate | 0.007 |
| vitamin B12 | 0.002 |
| vitamin D3 | 0.000001 |
| Riboflavin | 0.0008 |
| thiamin | 0.0006 |
| vitamin B6 | 0.0004 |
| folic acid | 0.0001 |
| vitamin K1 | 0.006 |
| biotin | 0.00002 |
| inositol | 0.03 |
| vitamin E acetate | 0.01 |
| taurine | 0.05 |
| L-carnitine | 0.001 |

### EXAMPLE 2

This example illustrates another embodiment of a nutritional product according to the present disclosure.

| **Description** | **kg per 100 kg** |
|---|---|
| carbohydrate, total | 24.7 |
| protein, total | 31.9 |
| fat, total | 39.3 |
| | |
| prebiotics | 3.6 |
| lactoferrin | 0.1 |
| calcium carbonate | 0.15 |
| ferrous sulfate | 0.03 |
| zinc sulfate | 0.01 |
| copper sulfate | 0.00025 |
| manganese sulfate | 0.0002 |
| sodium selenite | 0.00001 |
| choline bitartrate | 0.05 |
| ascorbic acid | 0.004 |
| sodium ascorbate | 0.04 |
| niacinamide | 0.007 |
| calcium pantothenate | 0.0005 |
| vitamin A palmitate | 0.0005 |
| vitamin D3 | 0.0002 |
| riboflavin | 0.0001 |
| thiamin | 0.00005 |
| vitamin B6 | 0.00005 |
| folic acid | 0.000067 |
| vitamin K1 | 0.00002 |
| vitamin E acetate | 0.008 |
| taurine | 0.02 |
| fish oil | 0.2 |
| B-glucan | 0.03 |

### EXAMPLE 3

This example illustrates one embodiment of ingredients that can be used to prepare the nutritional product according to the present disclosure.

| | |
|---|---|
| water | 872 ml |
| lactose | 65.6 mg |
| vegetable oil blend | 353.0 mg |
| nonfat milk evaporated | 34.0 mg |
| whey protein concentrate | 8.5 mg |
| galacto-oligosaccharide | 4.7 mg |
| casein | 3.5 mg |
| polydextrose | 2.4 mg |
| lactoferrin solution (10%) | 1.0 mg |
| single cell DHA and ARA oil blend | 0.94 mg |
| mono- and di-glycerides | 0.7 mg |
| calcium carbonate | 0.44 mg |
| calcium phosphate | 0.4 mg |
| potassium citrate | 0.4 mg |
| potassium chloride | 0.4 mg |
| soy lecithin | 0.4 mg |
| sodium chloride | 0.3 mg |
| potassium phosphate | 0.3 mg |
| choline chloride | 0.2 mg |
| magnesium oxide | 0.08 mg |
| calcium hydroxide | 0.08 mg |
| ferrous suflate | 0.07 mg |

### EXAMPLE 4

This example illustrates another embodiment of ingredients that can be used to prepare the nutritional product according to the present disclosure.

| | |
|---|---|
| water | 686 ml |
| reduced minerals whey | 215 mg |
| nonfat milk evaporated | 67 mg |
| vegetable oil blend | 33 mg |
| lactose | 17 mg |
| galacto-oligosaccharide | 4.7 mg |
| polydextrose | 2.4 mg |
| lactoferrin solution (10%) | 1.0 mg |
| single cell DHA and ARA oil blend | 0.9 mg |
| mono- and di-glycerides | 0.7 mg |
| calcium carbonate | 0.44 mg |
| calcium phosphate | 0.4 mg |
| potassium citrate | 0.4 mg |
| potassium chloride | 0.4 mg |
| soy lecithin | 0.4 mg |
| potassium phosphate | 0.3 mg |
| carrageenan | 0.3 mg |
| sodium citrate | 0.2 mg |
| choline chloride | 0.2 mg |
| magnesium oxide | 0.08 mg |
| calcium chloride | 0.08 mg |
| ferrous suflate | 0.07 mg |

Preferably, the nutritional composition is administered to a human and stimulates at least one type of innate immune cell in the human.

All references cited in this specification, including without limitation, all papers, publications, patents, patent applications, presentations, texts, reports, manuscripts, brochures, books, internet postings, journal articles, periodicals, and the like, are hereby incorporated by reference into this specification in their entireties. The discussion of the references herein is intended merely to summarize the assertions made by their authors and no admission is made that any reference constitutes prior art. Applicants reserve the right to challenge the accuracy and pertinence of the cited references.

Although preferred embodiments of the disclosure have been described using specific terms, devices, and methods, such description is for illustrative purposes only. The words used are words of description rather than of limitation. It is to be understood that changes and variations may be made by those of ordinary skill in the art without departing from the spirit or the scope of the present disclosure, which is set forth in the following claims. In addition, it should be understood that aspects of the various embodiments may be interchanged both in whole or in part. For example, while methods for the production of a commercially sterile liquid nutritional supplement made according to those methods have been exemplified, other uses are contemplated. Therefore, the spirit and scope of the appended claims should not be limited to the description of the preferred versions contained therein.

The invention is further described with reference to the following aspects:
1. A method for stimulating at least one type of innate immune cell in a human comprising administering to the human a nutritional composition comprising:
   a) a fat or lipid source;
   b) a protein source; and
   c) lactoferrin produced by a non-human source, wherein the lactoferrin has at least 48% homology with the amino acid sequence AVGEQELRKCNQWSGL at the HLf (349-364) fragment,
   such that administration of the nutritional composition stimulates at least one type of innate immune cell selected from the group consisting of macrophages, neutrophils, dendritic cells, and combinations thereof.
2. The method according to aspect 1, wherein the nutritional composition is administered to an infant or a child.
3. The method according to aspect 1, wherein the fat or lipid source is present at a level of about 3 g/100 kcal to about 7 g/100 kcal.
4. The method according to aspect 1, wherein protein source is present at a level of about 1 g/100 kcal to about 5 g/100 kcal.
5. The method according to aspect 1, wherein the lactoferrin is present at a level of at least about 10 mg/100 kcal.
6. The method according to aspect 5, wherein the lactoferrin is present at a level of about 70 mg/100 kcal to about 220 mg/100 kcal.
7. The method according to aspect 1, wherein the lactoferrin is selected from the group consisting of non-human lactoferrin, human lactoferrin produced by a genetically modified organism, and combinations thereof.
8. The method according to aspect 1, wherein the lactoferrin is stable and remains active under conditions under which human lactoferrin becomes unstable or inactive.
9. The method according to aspect 8, wherein the nutritional composition has been subject to pasteurization conditions.
10. The method according to aspect 1, wherein the nutritional composition further comprises a prebiotic composition comprising a compound selected from the group consisting of galactooligosaccharide, polydextrose, and combinations thereof.
11. The method according to aspect 1, wherein the nutritional composition comprises about 0.5 mg/100 kcal to about 5 mg/100 kcal of iron, including iron bound to lactoferrin.
12. The method according to aspect 1, wherein the nutritional composition further comprises about 5 mg/100 kcal to about 100 mg/100 kcal of at least one source of long chain polyunsaturated fatty acids.
13. The method according to aspect 1, further comprising the step of administering at least one probiotic.

## Claims

1. An infant formula comprising:
a) a fat or lipid source;
b) a protein source;
c) bovine lactoferrin, wherein the lactoferrin is present at a level of 70 mg/100 kcal to 220 mg/100 kcal; and
d) a prebiotic composition comprising a compound selected from the group consisting of galactooligosaccharide, polydextrose, and combinations thereof;
for use in a method for stimulating at least one type of innate immune cell in an infant comprising administering the infant formula to the infant such that administration of the infant formula stimulates at least one type of innate immune cell selected from the group consisting of macrophages, neutrophils, dendritic cells, and combinations thereof.

2. The infant formula for the use according to claim 1, wherein the infant formula has been subject to pasteurization conditions.

3. The infant formula for the use according to claim 1, wherein the infant formula comprises 0.5 mg/100 kcal to 5 mg/100 kcal of iron, including iron bound to lactoferrin.

4. The infant formula for the use according to claim 1, wherein the infant formula further comprises 5 mg/100 kcal to 100 mg/100 kcal of at least one source of long chain polyunsaturated fatty acids.

5. The infant formula for the use according to claim 1, further comprising at least one probiotic.

6. The infant formula for the use according to any one of the preceding claims, wherein the use if for stimulating the production of cytokines from macrophages and/or dendritic cells.

7. The infant formula for the use according to any one of the claims 1-6, wherein the use if for stimulating the respiratory burst capacity of neutrophils.
